# EUROPÄISCHE PATENTSCHRIFT

(11) **EP 1 610 729 B1**
(45) Veröffentlichungstag und Bekanntmachung des Hinweises auf die Patenterteilung: **18.05.2011**
(21) Anmeldenummer: 04722819.2
(22) Anmeldetag: 24.03.2004
(51) Int. Cl.: A61F 2/34

(54) **POLYLOCK-INSERT FÜR EIN KÜNSTLICHES HÜFTGELENK**
POLYLOCK INSERT FOR AN ARTIFICIAL HIP JOINT
PIECE RAPPORTEE DE TYPE POLYLOCK POUR ARTICULATION ARTIFICIELLE DE LA HANCHE

(30) Priorität: 25.03.2003 DE 10313413; 06.03.2004 DE 102004011046
(43) Veröffentlichungstag der Anmeldung: 04.01.2006
(73) Patentinhaber: CeramTec GmbH, 73207 Plochingen (DE)
(72) Erfinder: LEYEN, Stefan, 73776 Altbach (DE); BUNZ, Uwe, 72649 Wolfschlugen (DE)
(74) Vertreter: Uppena, Franz
(86) Internationale Anmeldenummer: PCT/EP2004/003093
(87) Internationale Veröffentlichungsnummer: WO 2004/084772

(56) Entgegenhaltungen:
- WO-A-01/05338
- GB-A- 2 365 343
- US-A- 4 068 324
- US-A- 5 916 269
- US-A- 6 162 257
- US-B1- 6 379 389

## Beschreibung

Die Erfindung betrifft eine Hüftgelenkprothese nach dem Oberbegriff des Anspruchs 1.

Ein künstliches Hüftgelenk besteht in der Regel aus einer Gleitschale, die direkt oder über einen Kunststoffmantel in eine äußere Metallschale eingesetzt ist. Diese Metallschale wird in den Beckenknochen implantiert. Die Kombination Gleitschale mit Kunststoffmantel wird auch als Sandwich-Insert bezeichnet.

In den Oberschenkelknochen wird hierzu ein Schaft implantiert, auf dem ein Kugelkopf angeordnet ist, der in der Gleitschale artikuliert.

Immer wieder kann es bei künstlichen Hüftgelenken zum Anschlagen des Kugelkopfschaftes an die Hüftgelenkspfanne kommen. Sind die Anschlagkräfte groß genug, kann dies zur Auflösung des mechanischen Hüftgelenkpfannenverbundes führen. Insbesondere Sandwich-Pfannensysteme sind hier gefährdet, da das überwiegen verwendete PE (Polyethylen) nur einen unzureichenden Widerstand gegen diese Anschlagkräfte aufbieten kann.

Sandwich-Inserts werden auf verschiedene Art und Weise hergestellt.

Bei einem System wird die keramische Gleitschale bzw. das Insert mit Kunststoff umspritzt, wobei an der Gleitschale Rücksprünge angeordnet sind. Nachteilig hieran sind die schlechteren Polyethylen (PE) - Eigenschaften, die durch die Erwärmung desselben resultieren. Ferner erfolgt ein Thermoschock für die keramische Gleitschale. Neben dem Umspritzaufwand durch die Spritzform und der Handhabung der heißen Teile ist der große Bauraumbedarf von Nachteil.

Bei einem alternativen System wird die Gleitschale durch eine konische Klemmung im Kunststoffmantel verankert, bei teilweisen geringen Festigkeiten des Verbundbauteils. Nachteilig ist auch hier der große Bauraumbedarf.

Bevorzugt wird auch das warme Einpressen der Gleitschale in den Kunststoffmantel verwendet. Hierbei treten jedoch teilweise zu geringe Festigkeiten des Verbundbauteils auf. Außerdem sind enge Toleranzen wegen der Pressverbindung zu beachten.

GB-A-2 365 343 Figur 1 zeigt eine Hüftgelenkprothese mit einer inneren Gleitschale aus Keramik die auf ihrer Aussenseite eine Strukturierung aufweist die aus umlaufenden konvexen Rillen besteht.

Der Erfindung liegt die Aufgabe zugrunde, eine Hüftgelenkprothese nach dem Oberbegriff des Anspruchs 1 so zu verbessern, dass eine hohe Kippfestigkeit bei kleinem Bauraumbedarf erreicht ist.

Erfindungsgemäß wird diese Aufgabe dadurch gelöst, dass die Gleitschale auf ihrer Außenseite eine Strukturierung aufweist, wodurch die Kipp- oder Umschlagfestigkeit wesentlich erhöht ist bei minimalem Bauraumbedarf, da die Strukturierung nahezu keine Bauraumvergrößerung erforderlich macht. Derartige Hüftgelenkprofhesen werden auch als Polylock-Inserts bezeichnet.

Die Strukturierung wird vorteilhafterweise mit großen Radien im Kerbgrund versehen um etwaige auftretende Kerbspannungen zu minimieren. Folgende Möglichkeiten einer Strukturierung sind denkbar:
- wellenförmige Vertiefungen oder
- halbkreisförmige Vertiefungen.

Hierbei soll der Kerbradius am Grund größer als 0,5 mm sein, um eine hohe Bauteilsicherheit zu erreichen.

Die wellenförmigen Vertiefungen sind bevorzugt auf der Außenseite der Gleitschale umlaufend angeordnet.

Die Gleitschale weist bevorzugt auf ihrer Außenseite eine sphärische oder eine abgestufte Bauform auf. Sphärische Bauformen haben einen sehr kleinen Bauraumbedarf.

In vorteilhafter Ausführungsform umklammert der Kunststoffmantel die Gleitschale an ihrem offenen Ende, wobei bevorzugt der auf der Oberseite der Gleitschale aufliegende Kragen des Kunststoffmantels nahezu die Hälfte des Oberrandes bedeckt.

Hergestellt wird das Sandwich-Insert bevorzugt durch Einpressen der Gleitschale in den Kunststoffmantel.

In bevorzugter Ausführungsform ist die Innenform der Gleitschale exzentrisch zur äußeren Form der Gleitschale angeordnet.

Die Abweichung zur Koaxialität (Exzentrizität) beträgt vorteilhafterweise mindestens 0,001 mm.

Weitere Merkmale der Erfindung ergeben sich aus den Figuren, die nachfolgend beschrieben sind. Es zeigt:
Fig. 1 ein erfindungsgemäßes Sandwich-Insert in sphärischer Bauform,
Fig. 2 ein erfindungsgemäßes Sandwich-Insert in abgestufter Bauform,
Fig. 3 eine wellenartige Strukturierung der Außenseite des Inserts und
Fig. 4 eine halbkreisförmige Strukturierung der Außenseite des Inserts.

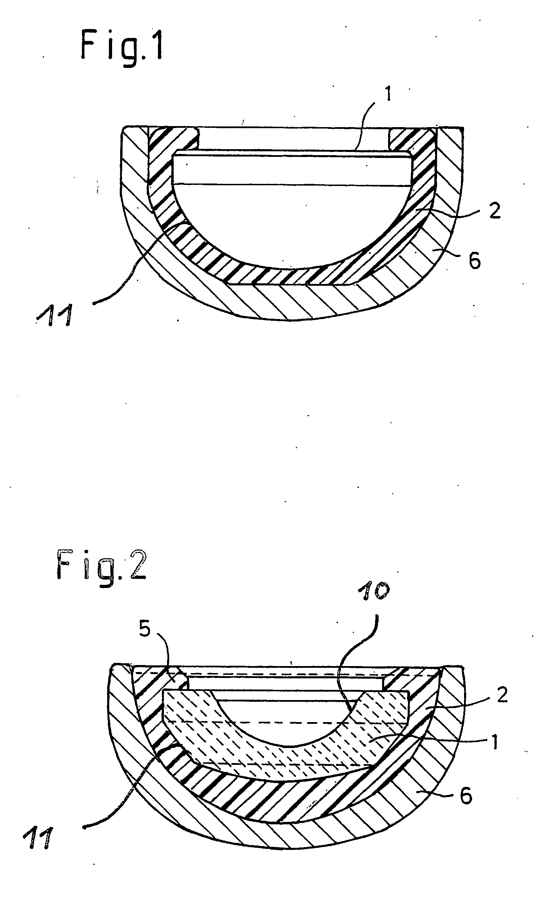

Fig. 1 zeigt ein Sandwich-Insert mit einer Gleitschale 1 aus Keramik in sphärischer Bauform. Bei der Herstellung wird diese Gleitschale 1 in den Kunststoffmantel 2 gepresst. Der Kunststoffmantel 2 besteht bevorzugt aus Polyethylen (PE). Dieses Sandwich-Insert ist in eine äußere Metallschale 6 eingesetzt.

Fig. 2 zeigt eine alternative Ausführungsform mit einer abgestuften Bauform der Gleitschale 1 auf ihrer Außenseite.

An ihrem offenen Ende umklammert der Kunststoffmantel 2 die Gleitschale 1, wodurch die Befestigung verbessert ist (vgl. Fig. 1). Der auf der Oberseite der Gleitschale 1 aufliegende Kragen 5 des Kunststoffmantels 2 bedeckt nahezu die Hälfte des Oberrandes.

Diese beiden in den Figuren 1 und 2 gezeigten Sandwich-Inserts zeigen nicht die erfindungsgemäße Strukturierung auf der Außenseite der Gleitschale 1.

In bevorzugter Ausführungsform der Erfindung ist die Innenform 10 der Gleitschale 1 exzentrisch zur äußeren Form 11 der Gleitschale 1 angeordnet, wobei die Abweichung zur Koaxialität (Exzentrizität) vorteilhafterweise mindestens 0,001 mm beträgt.

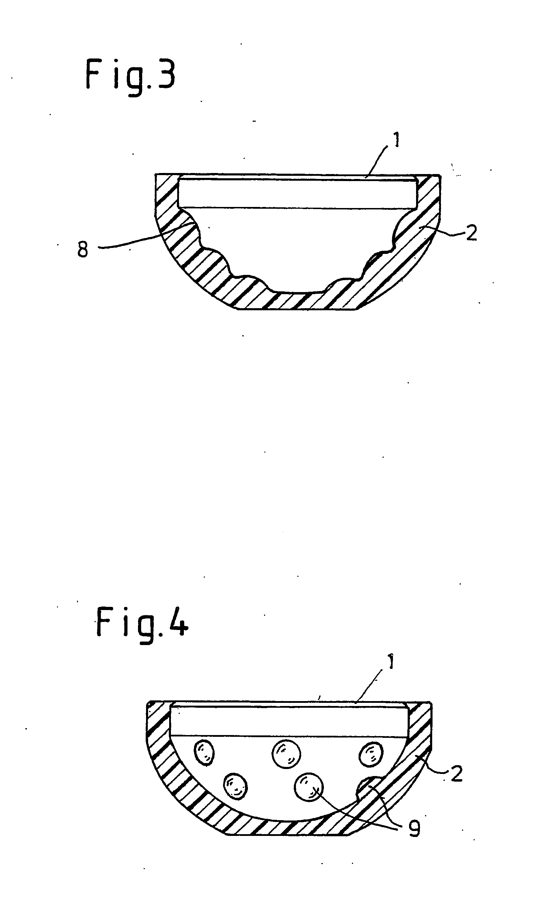

Figur 3 zeigt ein Sandwich-Insert, d. h. eine keramische Gleitschale 1 eingesetzt in einen Kunststoffmantel 2. Auf der Außenseite der Gleitschale 1 ist eine Strukturierung angeordnet, die aus im Schnitt einer wellenförmigen Vertiefung 8 besteht. Wichtig ist hierbei, dass diese Strukturierung mit großen Radien im Kerbgrund versehen ist, die größer als 0,5 mm betragen muss. Erst diese Radien im Kerbgrund bewirken eine gute Kipp- und Umschlagfestigkeit der Gleitschale 1 im Kunststoffmantel 2.

Die wellenförmige Vertiefung 8 ist umlaufend auf der Außenseite der Gleitschale 1 angeordnet.

Figur 4 zeigt eine Strukturierung, die aus halbkreisförmigen Vertiefungen 9 besteht. Auch hier sind die Vertiefungen mit Kerbradien größer als 0,5 mm am Kerbgrund versehen.

Das Sandwich-Insert, welches in den Figuren 1 bis 4 gezeigt ist, wird auch als Polylock-Insert bezeichnet und z. B. in eine äußere Metallschale 6 eingesetzt, die dann in einen Beckenknochen implantiert wird. Alternativ kann dieses Insert auch direkt in einen Beckenknochen mit Hilfe von Knochenzement implantiert werden.

In der Gleitschale 1 artikuliert ein hier nicht gezeigter Kugelkopf, der bevorzugt aus Keramik besteht. Dieser Kugelkopf ist auf einem Schaft befestigt, der im Oberschenkelknochen implantiert ist.

## Patentansprüche

1. Hüftgelenkprothese mit einer inneren Gleitschale (1) aus Keramik, die auf ihrer Außenseite von einem Kunststoffmantel (2) umschlossen ist, zum Einsetzen in eine äußere Metallschale (6) oder zur direkten Implantierung mit Hilfe von Knochenzement, wobei in der inneren Gleitschale (1) ein Kugelkopf artikulierbar ist, der auf einem Schaft angeordnet ist, der im Oberschenkelknochen
verankerbar ist, wobei die Gleitschale (1) auf ihrer Außenseite eine Strukturierung aufweist, **dadurch gekennzeichnet, dass** die Strukturierung mit großen Radien im Kerbgrund versehen ist und der Kerbradius am Kerbgrund größer als 0,5 mm beträgt.

2. Hoftgelenkprothese nach Anspruch 1, **dadurch gekennzeichnet, dass** die Strukturierung aus im Schnitt wellenförmigen Vertiefungen (8) besteht.

3. Hüftgelenkprothese nach Anspruch 2, **dadurch gekennzeichnet, dass** die im Schnitt wellenförmigen Vertiefungen (8) auf der Außenseite der Gleitschale (1) umlaufend angeordnet sind.

4. Hüftgelenkprothese nach einem der Ansprüche 1 bis 3, **dadurch gekennzeichnet, dass** die Strukturierung aus halbkreisförmigen Vertiefungen (9) besteht.

5. Hoftgelenkprothese nach einem der Ansprüche 1 bis 4, **dadurch gekennzeichnet, dass** die Gleitschale (1) auf ihrer Außenseite eine sphärische oder eine abgestufte Bauform aufweist.

6. Hoftgelenkprothese nach einem der Ansprüche 1 bis 5, **dadurch gekennzeichnet, dass** der Kunststoffmantel (2) die Gleitschale (1) an Ihrem offenen Ende umklammert.

7. Hüftgelenkprothese nach Anspruch 6, **dadurch gekennzeichnet, dass** ein auf der Oberseite der Gleitschale (1) aufliegende Kragen (5) des Kunststoffmantels (2) nahezu die Hälfte des Oberrandes bedeckt.

8. Hüftgelenkprothese nach einem der Ansprüche 1 bis 7, **dadurch gekennzeichnet, dass** die Gleitschale (1) durch Einpressen in den Kunststoffmantel (2) mit diesem verbunden ist.

9. Hüftgelenkprothese nach einem der Ansprüche 1 bis 8, **dadurch gekennzeichnet, dass** die Innenform (10) der Gleitschale (1) exzentrisch zur äußeren Form (11) der Gleitschale (1) angeordnet ist.

10. Hüftgelenkprothese nach Anspruch 9, **dadurch gekennzeichnet, dass** die Abweichung zur Koaxialität (Exzentrizität) mindestens 0,001 mm beträgt

## Claims

1. A hip joint prosthesis having an inner sliding cup (1) made of ceramic material that is surrounded on its outside by a plastics covering (2), for insertion into an outer metal cup (6) or for direct implantation with the aid of bone cement, wherein a ball head that is arranged on a shaft, which can be anchored in the femur, can be articulated in the inner sliding cup (1), wherein the sliding cup (1) has a structuring on its outside, **characterised in that** the structuring is provided with large radii in the notch base, and the notch radius at the notch base amounts to more than 0.5 mm.

2. A hip joint prosthesis according to claim 1, **characterised in that** the structuring consists of depressions (8) that are undulating in section.

3. A hip joint prosthesis according to claim 2, **characterised in that** the depressions (8) that are undulating in section are circumferentially arranged on the outside of the sliding cup (1).

4. A hip joint prosthesis according to one of claims 1 to 3, **characterised in that** the structuring consists of semicircular depressions (9).

5. A hip joint prosthesis according to one of claims 1 to 4, **characterised in that** the sliding cup (1) has on its outside a spherical or stepped structural form.

6. A hip joint prosthesis according to one of claims 1 to 5, **characterised in that** the plastics covering (2) embraces the sliding cup (1) at its open end.

7. A hip joint prosthesis according to claim 6, **characterised in that** a collar (5) of the plastics covering (2) that rests on the upper side of the sliding cup (1) covers almost half of the upper edge.

8. A hip joint prosthesis according to one of claims 1 to 7, **characterised in that** the sliding cup (1) is connected to the plastics covering (2) by being pressed into the plastics covering (2).

9. A hip joint prosthesis according to one of claims 1 to 8, **characterised in that** the inner form (10) of the sliding cup (1) is arranged eccentrically in relation to the outer form (11) of the sliding cup (1).

10. A hip joint prosthesis according to claim 9, **characterised in that** the variation with respect to the coaxial (the eccentricity) amounts to at least 0.001 mm.

## Revendications

1. Prothèse de la hanche, comprenant une cupule (1) intérieure en céramique qui est entourée sur sa face externe d'une enveloppe en matière plastique (2), en vue de l'insertion dans une coque métallique (6) extérieure ou de l'implantation directe à l'aide de ciment à os, une tête sphérique, qui est disposée sur une tige ancrée dans l'os fémoral, pouvant être articulée dans la cupule (1) intérieure, la cupule (1) présentant une structuration sur sa face externe, **caractérisée par le fait que** la structuration est dotée de grands rayons en fond d'encoche, et le rayon d'encoche dans le fond d'encoche est supérieur à 0,5 mm.

2. Prothèse de la hanche selon la revendication 1, **caractérisée par le fait que** la structuration est constituée de creux (8) à section ondulée.

3. Prothèse de la hanche selon la revendication 2, **caractérisée par le fait que** les creux (8) à section ondulée sont disposés de manière périphérique sur la face externe de la cupule (1).

4. Prothèse de la hanche selon une des revendications 1 à 3, **caractérisée par le fait que** la structuration est constituée de creux (9) semi-circulaires.

5. Prothèse de la hanche selon une des revendications 1 à 4, **caractérisée par le fait que** la cupule (1) présente une forme sphérique ou étagée sur sa face externe.

6. Prothèse de la hanche selon une des revendications 1 à 5, **caractérisée par le fait que** l'enveloppe en plastique (2) enserre la cupule (1) à son extrémité ouverte.

7. Prothèse de la hanche selon la revendication 6, **caractérisée par le fait qu'**un collet (5) de enveloppe en plastique (2), qui repose sur la face supérieure de la cupule (1), recouvre presque la moitié du bord supérieur.

8. Prothèse de la hanche selon une des revendications 1 à 7, **caractérisée par le fait que** la cupule (1) est reliée à l'enveloppe en plastique (2) en étant pressée dans celle-ci.

9. Prothèse de la hanche selon une des revendications 1 à 8, **caractérisée par le fait que** la forme intérieure (10) de la cupule (1) est disposée de façon excentrée par rapport à la forme extérieure (11) de la cupule (1).

10. Prothèse de la hanche selon la revendication 9, **caractérisée par le fait que** l'écart de coaxialité (excentricité) est au moins de 0,001 mm.
